# EUROPEAN PATENT APPLICATION

(11) **EP 2 939 636 A1**
(43) Date of publication of application: **04.11.2015**
(21) Application number: 13866680.5
(22) Date of filing: 19.11.2013
(51) Int. Cl.: A61F 2/16

(54) **LENTICULAR CAPSULE-EXPANDING DEVICE**

(30) Priority: 26.12.2012 JP 2012282287
(71) Applicant: Frontier Vision Co., Ltd., Hyogo 663-8111 (JP)
(72) Inventor: Akura, Junsuke, Higashimuro-gun Wakayama 649-3510 (JP)
(74) Representative: Viering, Jentschura & Partner Patent- und Rechtsanwälte
(86) International application number: PCT/JP2013/081177
(87) International publication number: WO 2014/103564

(57) **Abstract**

The lenticular capsule-expanding device (1) is set inside a lens capsule in which the anterior capsule has been sectioned in cataract surgery, etc. and is equipped with: a circular front support section (11), which is provided in a form that connects the inner surface of the anterior capsule (Sf); a circular rear support section (12) that is provided on the rear side of the front support section (11) in a form that contacts the inner surface of the posterior capsule (Sb) while facing the front support section (11); and a connecting section (13) for connecting the rim of the front support section (11) to the rim of the posterior support section (12) in a form that has a biasing force in the direction that separates the front section (11) from the rear support section (12). As a result of the biasing force of the connecting section (13), the front support section (11) presses the inner surface of the anterior capsule (Sf) and the rear support section (12) presses the inner surface of the posterior capsule (Sb).

## Description

### TECHNICAL FIELD

The present invention relates to a lenticular capsule-expanding device to be arranged in a lens capsule in which an anterior capsule is cut in a cataract surgery, etc.

### BACKGROUND ART

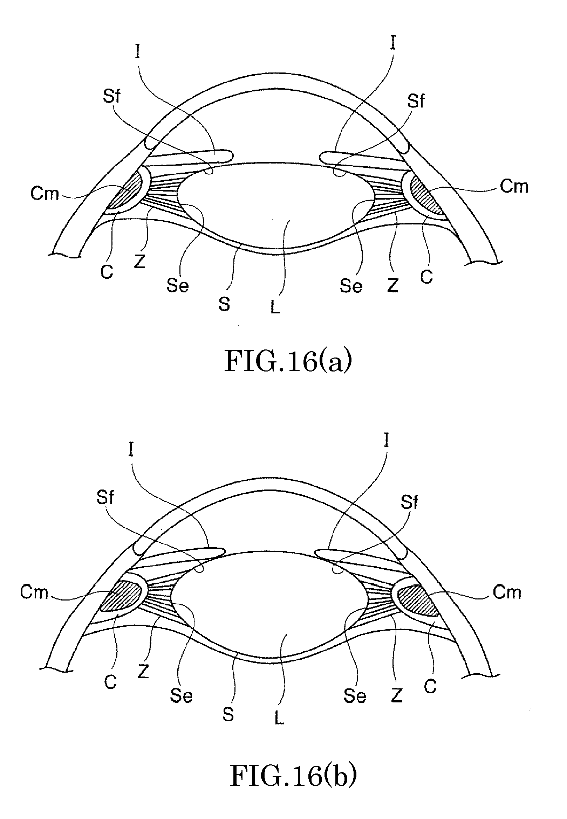

Normally, focusing of a human eye (hereinafter simply referred to as "focusing") is performed by changing a thickness of a crystalline lens. As shown in Fig. 16, a crystalline lens L is a transparent member of a convex shape having a diameter of about 9 to 10 mm and a thickness of about 4 to 5 mm and exerting a lens function, and is fixed to a ciliary body C via a Zinn's zonule Z in a manner as to be arranged behind the iris I with the lens encapsulated by a lens capsule S.

Concrete focusing mechanism will be explained as follows. For example, in the case of seeing a far distance, as shown in Fig. 16(a), the ciliary muscle Cm of the ciliary body C is in a relaxed state, and therefore the ciliary body C is in a position retracted in a direction apart from the lens capsule S. In this state, a relatively strong tension is applied to the Zinn's zonule Z positioned between the ciliary body C and the equator Se of the lens capsule S. As a result, the equator Se of the lens capsule S is pulled radially outward to cause deformation of the lens L so that the thickness of the crystalline lens L in the lens capsule S decreases. In accordance with this deformation, the thickness of the crystalline lens L in the lens capsule S decreases, focusing at the time of seeing a far distance is performed.

On the other hand, in the case of trying to see a near object, as shown in Fig. 16(b), the ciliary muscle Cm of the ciliary body C contracts to cause protrusion of the ciliary body C centripetally (in a direction toward the equator Se of the lens capsule S), resulting in a movement of the ciliary body C in a direction approaching the lens capsule S. As a result, the tensile force of the Zinn's zonule Z decreases, causing deformation of the crystalline lens L so that the thickness of the crystalline lens L increases by the elastic force inherent in the crystalline lens L. Thus, focusing at the time of seeing a near distance is performed.

As explained above, in accordance with contraction and relaxation of the ciliary muscle Cm, the thickness of the crystalline lens L is changed to thereby perform focusing by causing refraction of the light entered into an eye. In this focusing mechanism, it is known that the contraction function and relaxation function by the ciliary muscle Cm of the ciliary body C are kept well even at an old age in the same manner as at a young age. On the other hand, it is also known that the contents of the crystalline lens L and the lens capsule S become hardened to lose the flexibility, resulting in difficulty of thickness changes of the crystalline lens L, which loses the force of voluntarily adjusting the focal point (hereinafter referred to as "focusing ability") when seeing a near distance from the state of seeing a far distance (which will be called presbyopia).

By the way, among diseases on a crystalline lens as mentioned above, there is a disease called "cataract" in which a crystalline lens becomes cloudy mainly caused by advancing age. Many patients have cataract surgeries for treating their cataracts. In the cataract surgery, normally, a method is employed in which a circular hole is formed in the anterior capsule, and the contents of the cloudy crystalline lens are removed through the circular hole by an ultrasonic crystalline lens emulsion suction method to remain only a transparent lens capsule in a cut and opened state, and an intraocular lens is inserted into the lens capsule. The cataract surgery of this method has been currently applied to patients of more than 1 million in Japan every year and patients of more than 3 million in the United States of America every year.

However, a single focus lens is generally made of a material such as a PMMA (Poly Methyl Methacrylate), silicon, acrylic, etc., which is not capable of changing a thickness of the single focus lens itself. For this reason, it was inevitable to avoid the loss of focusing ability after the surgery. On the other hand, there are also known a refractive type multifocal lens in which sections concentrically different in refractive power are formed in an optical section, a multifocal lens in which a refractive multifocal lens is provided with a structure causing an optical diffraction phenomenon in an optical section so that light to be entered into an eye is taken in a dispersed manner for a far distance use and a near distance use (in some cases, further for an intermediate distance use). However, in such multifocal intraocular lens, there are reports that patients complain of a halo phenomenon in which an object looks with light rings, a glare in which an object looks glistening, insufficient eyesight, or insufficient contrast sensitivity. Thus, such multifocal intraocular lenses could not have sufficiently satisfied patient's requests.

In recent years, as an intraocular lens capable of exerting an adjustment function by a method different from the above-mentioned method, there is known an intraocular lens called "accommodative intraocular lens" provided with an optical section made of a convex lens and two connection arms of a joint connection type to be arranged in a manner as to come into contact with an inner side of a lens capsule equator so that adjustment is performed by moving the optical section back and forth (see the following Patent Document 1). In this intraocular lens, the connection arm is attached to the optical section at a first position on the connection arm, and works harmoniously with the ciliary muscle of the ciliary body or the Zinn's zonule at a second positon on the connection arm.

On the other hand, there have been proposed a number of ring-shaped lenticular capsule-expanding devices used to expand a lens capsule before inserting an intraocular lens at the time of cataract surgery. Two types of these ring-shaped lenticular capsule-expanding devices are used depending on the purpose.

One of the ring-shaped lenticular capsule-expanding devices is called "Capsular Tension Ring," which is an open ring formed into a C-shape. This ring is inserted inside the lens capsule equator in which a Zinn's zonule is weakened or broken to expand the lens capsule equator radially outward to form a round shape.

The other of the ring-shaped lenticular capsule-expanding devices is called "Equator ring," which is an O-shaped ring. This ring is a closed type ring (continuous ring) having a cross-section with sharp edges such as a square cross-section and a rather large thickness. This ring is arranged at an inner side of the lens capsule equator to form a strong bent section of the lens capsule to thereby prevent growth and approach of the lens epidermal cell inside.

### PRIOR ART DOCUMENT

### PATENT DOCUMENT

Patent Document 1: Japanese Unexamined Patent Application Publication No. 11-47168

### SUMMARY OF THE INVENTION

### Problems to be solved by the invention

However, a focusing adjustment function of a human eye is exerted based on contraction and relaxation of a ciliary muscle of a ciliary body, and therefore, in order to move an optical section of an intraocular lens in a front-back direction, it is required to cause deformation of a lens capsule by accurately transmitting slight contraction/relaxation of the ciliary muscle of the ciliary body. To do it, it is important that a Zinn's zonule, which transmits contraction and relaxation of a ciliary muscle of a ciliary body to a lens capsule, has continuous tonus of moderate strength.

In this regard, a conventional intraocular lens does not act so that a Zinn's zonule has continuous tonus of moderate strength. Therefore, it is difficult to accurately convert slight contraction and relaxation of a ciliary muscle of a ciliary body into movements of an optical section in a front-back direction. This in turn cannot accurately exert the adjustment function of the intraocular lens.

Similarly, the ring-shaped lenticular capsule-expanding device can adjust a position of a lens capsule equator and form a strong bent section in a lens capsule, but does not cause continuous tonus of moderate strength in a Zinn's zonule, but rather weakens the tonus of the Zinn's zonule by outwardly expanding the lens capsule equator. For this reason, slight contraction and relaxation of a ciliary muscle of a ciliary body cannot be transmitted accurately, which prevents accurate exertion of the adjustment function of the intraocular lens.

Further, in a conventional intraocular lens and ring-shaped lenticular capsule-expanding device, the connection arm and the ring-shaped lenticular capsule-expanding device itself are fixed in a manner as to be in contact with the inner side of the lens capsule equator. This causes adhesion of an anterior capsule and a posterior capsule of a crystalline lens, resulting in atresia of the lens capsule equator. When hydatoid always flows to the lens capsule equator, after-cataract unlikely occurs. Therefore, in recent years, it has been found that hydatoid has a function of controlling growth of a lens epidermal cell. However, in these conventional intraocular lenses, the lens capsule equator is not exposed to hydatoid, resulting in growth of lens epidermal cell at the lens capsule equator, which is in a state in which after-cataract likely occurs.

When after-cataract occurs, the following problems occur. That is, a central portion of the lens capsule becomes turbid or cloudy, causing difficulty in passing light, which in turn results in poor visual acuity. The equator of the crystalline lens adheres in the front-back direction, causing fibroplasia, which results in hardening of the equator. As a result, the joint connection type arm of the intraocular lens is fixed by fibers and becomes immovable, which prevents exertion of the adjustment function of the intraocular lens.

The present invention was made in view of the aforementioned problems, and aim to provide a lenticular capsule-expanding device in which a Zinn's zonule can accurately transmits slight contraction and relaxation of a ciliary muscle of a ciliary body to a lens capsule.

### Means for Solving the Problems

The present invention relate to a lenticular capsule-expanding device to be arranged in a lens capsule in which an anterior capsule is cut in a cataract surgery, etc., to attain the aforementioned objects. The lenticular capsule-expanding device includes a front supporting section to be arranged in a manner as to come into contact with an inner surface of the anterior capsule, the front supporting section allowing light to pass through rearward, a rear supporting section to be arranged in a manner as to come into contact with an inner surface of a posterior capsule, the rear supporting section being arranged so as to be opposed to the front supporting section and allowing light to pass through rearward, and a connecting section that connects the front supporting section and the rear supporting section in a manner as to have an urging force for separating the front supporting section and the rear supporting section in a separating direction.

According to this device, by the urging force of the connecting section, the front supporting section presses against the inner surface of the anterior capsule and the rear supporting section presses against the inner surface of the posterior capsule. Therefore, the peripheral portion of the lens capsule equator is stretched and expanded in the front-back direction, which expands the lens capsule equator and at the same time the lens capsule equator moves centripetally to reduce the diameter of the lens capsule equator. By this, the Zinn's zonule is pulled in both directions, i.e., in a direction toward the lens capsule side and in a direction toward the ciliary body side, which provides continuous tonus of moderate strength to the Zinn's zonule. Therefore, the Zinn's zonule can accurately transmit slight contraction and relaxation of the ciliary muscle of the ciliary body to the lens capsule.

Further, when the front supporting section is formed into an open state like a ring shape, hydatoid flows into the lens capsule from the cut portion of the anterior capsule through between the front supporting section and the connecting section. Thus, the lens capsule equator comes into contact with and is exposed to the hydatoid. This inhibits growth and fibrillization of the lens epidermal cell of the lens capsule equator, and therefore occurrence of after-cataract can be prevented.

Further, it is preferable that the connecting section has an urging force corresponding to a tensile force of a Zinn's zonule to be generated at a time of contraction or relaxation of a ciliary muscle of a ciliary body. In this case, a tonus of more moderate strength can be continuously applied to the Zinn's zonule.

Further, it is preferable that the connecting section includes a plurality of connecting members arranged at intervals in a peripheral direction of the front supporting section and the rear supporting section. In this case, by the urging force of the plurality of connecting members, the anterior capsule and the posterior capsule can be effectively stretched and extended along the entire periphery to open the lens capsulate equator. As a result, a tonus of moderate strength can be applied to the Zinn's zonule. Further, since the lens capsule equator is opened along the entire periphery, hydatoid can be assuredly flowed to the lens capsule equator, which in turn can more assuredly inhibit growth and fibrillization of the lens epidermal cell in the lens capsule equator Se.

Further, it is preferable that the connecting section bends radially outward of the front supporting section and the rear supporting section when the front supporting section and the rear supporting section move in an approaching direction. According to this, while applying a moderate urging force to the front supporting section and the rear supporting section, the front supporting section and the rear supporting section can be moved in the approaching/separating direction.

Further, it is preferable that the connecting section includes a previously formed bending portion capable of being bent radially outward of the front supporting section and the rear supporting section. According to this, based on the previously formed bending portion, the connecting section can be assuredly bent in a manner as to expand radially outward. Further, by engaging the intraocular lens with the inner side of the bending portion, the intraocular lens can be stably arranged.

Further, it is preferable that the connecting section is provided with a bending portion previously formed in a manner as to be bent. According to this, based on the previously bent bending portion, the connecting section can be more assuredly bent in a manner as to expand radially outward. Further, by engaging the intraocular lens with the inner side of the bending portion which is bent, the intraocular lens can be more assuredly arranged.

Further, it is preferable that the bending portion of the connecting section is provided at a position closer to the rear supporting section than the front supporting section. According to this, the front supporting section becomes more movable with respect to the rear supporting section in the front-back direction. Further, the position of the optical section of the present lens at the time of adjustment (at the time of seeing a far distance) can be positioned rearward, which can increase the forward moving amount at the time of adjustment (at the time of seeing a near distance).

Further, it is preferable that the connecting section is provided with an engaging portion for engaging an intraocular lens at an inner side of the bending portion. According to this, by engaging the intraocular lens with the engaging portion of the connecting section, the intraocular lens can be arranged more stably in the lenticular capsule-expanding device.

Further, it is preferable that the connecting section is provided with a control portion for controlling bending of the bending portion so as not to exceed a predetermined degree of bending. According to this, the bending portion is controlled so as not to be bent beyond the predetermined degree and therefore the front supporting section and the rear supporting section are separated by a certain distance or more, which in turn can more assuredly inhibit growth and fibrillization of the lens epidermal cell in the lens capsule equator Se after surgery.

Further, it is preferable that at least one the front supporting section and the rear supporting section is provided with a plurality of elastic protrusions protruded radially outward of an outer peripheral portion in a radial fashion. By this, the peripheral portion of the lens capsule equator Se is further stretched and expanded in the front-back direction to thereby further expand the lens capsule equator Se, which in turn can more assuredly inhibit growth and fibrillization of the lens epidermal cell in the lens capsule equator Se after surgery.

Further, it is preferable that at least one of the front supporting section and the rear supporting section is provided with at least one of a groove and a through-hole at a portion which comes into contact with an anterior capsule or a posterior capsule of a crystalline lens. With this, hydatoid flows to the portion where the front supporting section and the anterior capsule contact and the portion where the rear supporting section and the posterior capsule contact, via the groove and/or the through-hole. This inhibits growth of the lens epidermal cell at the contact portion to thereby prevent occurrence of after-cataract and also prevent anterior capsule contraction occurred followed by the after-cataract.

Further, it is preferable that at least one of the front supporting section and the rear supporting section is formed into a ring shape having an opening at its center. With this, the corrective lens for correcting the power of the intraocular lens can be detachably attached to the ring-shaped opening of the front supporting section and/or the rear supporting section, and therefore the corrective lens can be arbitrarily attached or replaced with another one.

Further, it is preferable that the front supporting section is provided with a plurality of cut-out portions extending radially outward from an inner peripheral portion in a radial fashion. With this, the inner peripheral portion of the front supporting section become more easily movable corresponding to the movement of the inner peripheral portion of the lens capsule which most moves by the adjustment. For this reason, when the connecting section is connected to the inner peripheral portion of the front supporting section, in accordance with the movement of the inner peripheral portion of the front supporting section, the bending degree of the connecting section changes. Therefore, it becomes possible to largely move the optical section of the intraocular lens between the front supporting section and the rear supporting section in the front-back direction.

Further, it is preferable that it further includes a second connecting section which connects the front supporting section and the rear supporting section in a manner as to have an urging force for separating the front supporting section and the rear supporting section in a separating direction, the second connecting section being formed into a shape extending along an inner peripheral surface of an equator of a lens capsule. By the urging force of the second connecting section, the front supporting section presses against the inner surface of the anterior capsule and the rear supporting section presses the inner surface of the posterior capsule. With this, together with or in place of the connecting section used to move mainly the intraocular lens, by the urging force of the second connecting section, the front supporting section and the rear supporting section can press against the anterior capsule and the posterior capsule of the lens capsule. Further, the second connecting section is formed into a shape extending along the inner peripheral surface of the equator of the lens capsule, and therefore the present device can be stably arranged in the lens capsule.

Further, it is preferable that an intraocular lens is arranged between the front supporting section and the rear supporting section. With this, the lens capsule device and the intraocular lens work together, and therefore the adjustment function of the intraocular lens can be exerted with high degree of accuracy.

Further, it is preferable that the intraocular lens includes an optical section made of a lens and a plurality of arm sections provided at a peripheral portion of the optical section, one end portion of each arm section is movably connected to a peripheral portion of the optical section, and the other end portion of each arm section is engaged with the connecting section, when the other end portion of the arm section is moved in the approaching/separating direction depending on a degree of expansion of the connecting section radially outward, the optical section to which the one end portion of the arm section is attached moves between the front supporting section and the rear supporting section in a front-back direction. With this, when the front supporting section and the rear supporting section move in the approaching direction between the front supporting section and the rear supporting section and the degree of radially outward expansion of the connecting section increases, the other end portions of the arm sections of the intraocular lens move in the separating direction, and in accordance with the movement, the optical section of the intraocular lens can move rearward. Further, when the front supporting section and the rear supporting section move in the separating direction and the degree of radially outward expansion of the connecting section decreases, the other end portions of the arm sections of the intraocular lens move in the approaching direction, and in accordance with the movement, the optical section of the intraocular lens can move forward. As explained above, by working the lenticular capsule-expanding device and the intraocular lens together, the adjustment function of the intraocular lens can be exerted effectively.

Further, it is preferable that the one end portion of the arm section connected to a peripheral portion of the optical section and the other end portion of the arm section to be engaged with the connecting section are arranged on opposite sides with respect to a center of the optical section. With this, the length/distance of the arm section from one end portion to the other end portion becomes longer and the movement of the other end portions of the arm sections in the approaching direction can be effectively converted into the movement of the optical section in the front-back direction. Therefore, even by slight movements of a lens capsule in accordance with slight contraction and relaxation of a ciliary muscle of a ciliary body, the adjustment function of the intraocular lens can be effectively exerted.

### BRIEF EXPLANATION OF THE DRAWINGS

Fig. 1 is a perspective view of a lenticular capsule-expanding device according to a first embodiment of the present invention.
Fig. 2(a) is a plan view of the lenticular capsule-expanding device of Fig. 1, and Fig. 2(b) is a bottom view of the lenticular capsule-expanding device.
Fig. 3(a) is a side view of the lenticular capsule-expanding device of Fig. 1, and Fig. 3(b) is a cross-sectional side view of the lenticular capsule-expanding device.
Fig. 4(a) is a plan view of an intraocular lens according to a first embodiment, and Fig. 4(b) is a side view of the intraocular lens according to the first embodiment.
Figs. 5(a) to 5(c) are show movements of the intraocular lens of Fig. 4, and Figs. 5(d) to 5(f) show movements of a conventional intraocular lens.
Fig. 6 is a perspective view of the lenticular capsule-expanding device in which the intraocular lens is arranged therein.
Fig. 7 is a side cross-sectional view showing movements of the lenticular capsule-expanding device shown in Fig. 1 and the intraocular lens shown in Fig. 4 at the time of focusing
Fig. 8 is a side view showing a principal portion of a lenticular capsule-expanding device according to a second embodiment.
Fig. 9 is a side view showing a principal portion of a lenticular capsule-expanding device according to a third embodiment.
Fig. 10 is a side view showing a principal portion of a lenticular capsule-expanding device according to a fourth embodiment.
Fig. 11 (a) is a side view of the device according to a fifth embodiment, and Fig. 11(b) is a top view thereof.
Fig. 12 is a plan view of the device according to a sixth embodiment.
Fig. 13(a) is a perspective view of the device according to a seventh embodiment, and Fig. 13(b) is a cross-sectional view thereof.
Fig. 14(a) is a top view of the device according to an eighth embodiment, and Fig. 14(b) is a side view thereof.
Fig. 15 is a cross-sectional view of the device shown in Fig. 14.
Fig. 16 is a cross-sectional side view of a human eye showing movements at the time of focus adjustments

### EMBODIMENTS FOR CARRYING OUT THE INVENTION

### <First Embodiment>

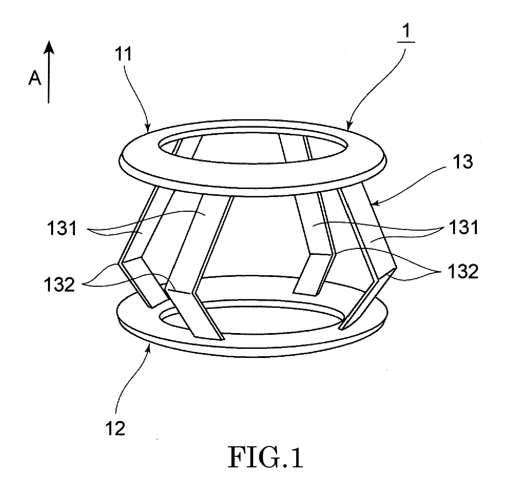

Next, a lenticular capsule-expanding device (hereinafter may be simply referred to as "the present device") and an intraocular lens (hereinafter may be simply referred to as "the present lens") to be arranged inside the present device according to the present invention will be explained with reference to Figs. 1 to 7. The following explanation will be made, assuming that the arrow direction A shown in figures denotes a direction toward a "front" side and the opposite direction denotes a direction toward a "rear/back" side.

### [Structure of Device]

As shown in Figs. 1 to 7, the present device 1 is configured to be arranged in a lens capsule S in which the anterior capsule Sf was cut in a surgery such as a cataract surgery, etc. The present device 1 is, as shown in Fig. 1, equipped with a front supporting section 11 to be positioned at a front side in the lens capsule S, a rear supporting section 12 to be positioned at a rear side in the lens capsule S, and a connecting section 13 which connects a peripheral portion of the front supporting section 11 and a peripheral portion of the rear supporting section 12.

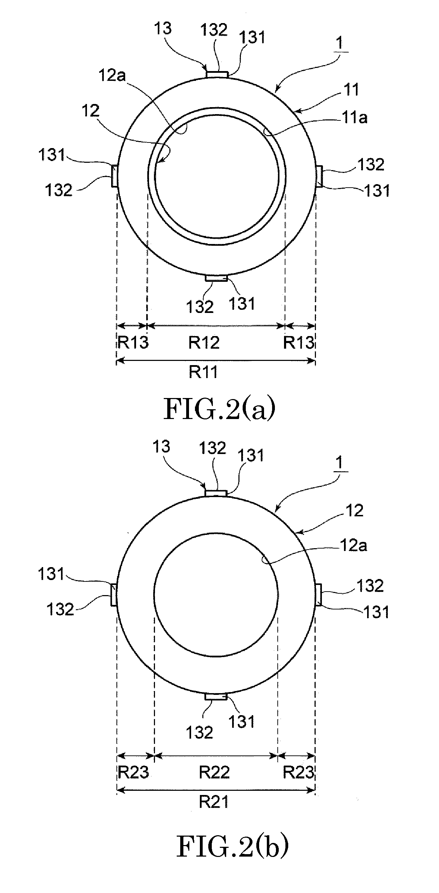

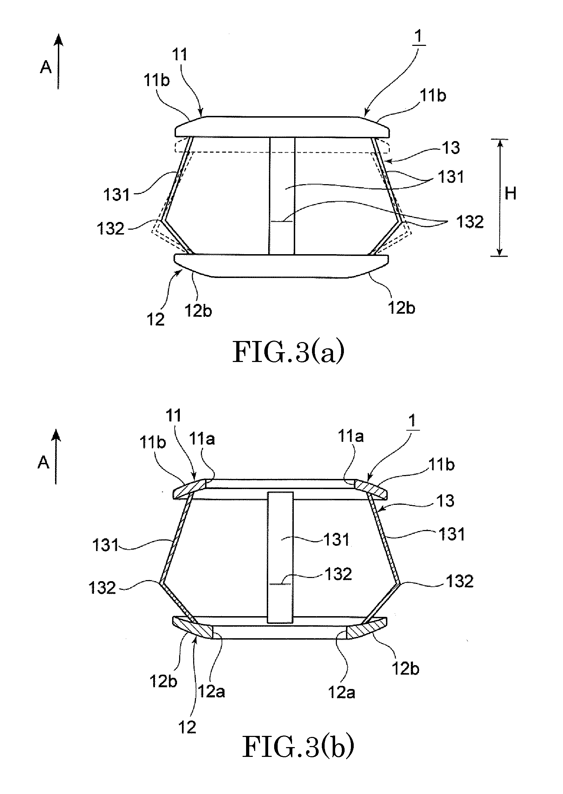

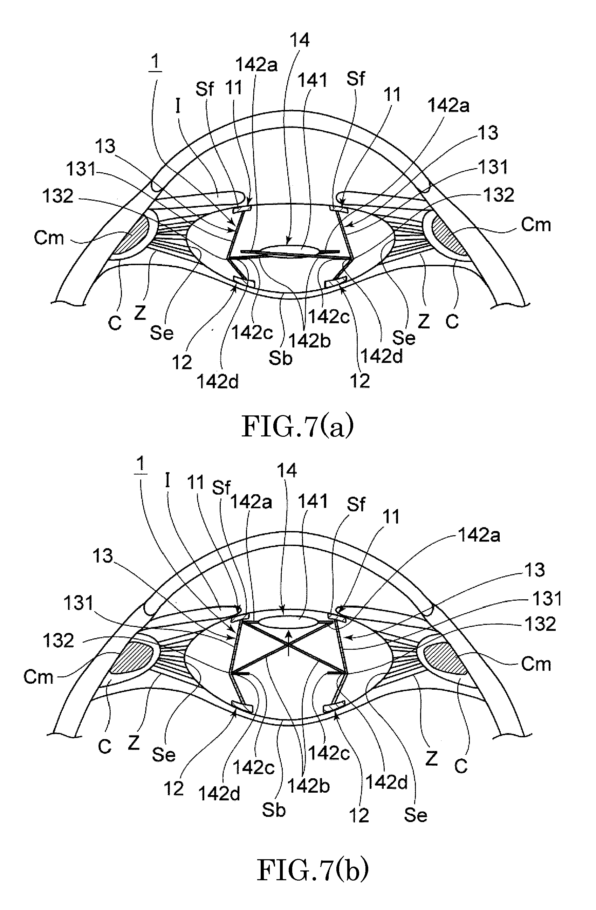

The front supporting section 11 is, as shown in Fig. 2(a), an elastic member formed into a circular/ring shape and having an opening 11a in the center thereof. This front supporting section 11 is provided with, as shown in Fig. 3, an inclined surface 11b which is formed on a front surface side and inclined rearward as it advances from a radially inward end portion of the front supporting section 11 toward a radially outward end portion thereof. For this reason, as shown in Fig. 7, the front supporting section 11 is arranged in a manner as to come into contact with an inner surface of an anterior capsule Sf of a lens capsule S by a cataract surgery, etc. Therefore, when the front supporting section 11 is in contact with the anterior capsule Sf, the inclined surface 11b can reduce the contact load to the anterior capsule Sf. Further, the front supporting section 11 is made of an elastic material, and therefore slightly deforms depending on a force received from the anterior capsule Sf, which further can reduce the contact load between the front supporting section 11 and the anterior capsule Sf.

Further, based on the fact that a conventional crystalline lens has a diameter of about 9 to 10 mm and a thickness of about 4 to 5 mm, as shown in Fig. 2(a), the front supporting section 11 is formed such that a diameter R11 of the outer periphery is 7.0 mm, a diameter R12 of the opening 11a is 5.0 mm, a width R13 of the inclined surface 11b which comes into contact with the anterior capsule Sf is 1.2 mm, and a thickness is 0.15 to 0.4 mm.

As shown in Fig. 2(b), in the same manner as in the front supporting section 11, the rear supporting section 12 is an elastic member formed into a circular/ring shape and having an opening 12a at the center thereof, and is arranged in a manner as to be opposed in parallel to the front supporting section 11 on the rear side thereof. This rear supporting section 12 is provided with, as shown in Fig. 3, an inclined surface 12b which is formed on a rear surface side and inclined frontward as it advances from a radially inward end portion of the front supporting section 11 toward a radially outward end portion thereof. For this reason, as shown in Fig. 7, the rear supporting section 12 is arranged in a manner as to come into contact with an inner surface of a posterior capsule Sb of a lens capsule S. Therefore, when the rear supporting section 12 is in contact with the posterior capsule Sb, the inclined surface 12b can reduce the contact load to the posterior capsule Sb. Further, since the rear supporting section 12 is made of an elastic material, the rear supporting section 12 slightly deforms depending on a force received from the posterior capsule Sb, which further can reduce the contact load between the rear supporting section 12 and the posterior capsule Sb. Further, the thickness of the rear supporting section 12 is formed so that the thickness is reduced as it advances from the radially inward side to the radially outward side.

Further, this rear supporting section 12 is formed based on the size of a standard crystalline lens, as shown in Fig. 2(b), so that the diameter R21 of the outer peripheral edge is 7.0 mm, the diameter R22 of the opening 12a is 4.5 mm, the width R23 of the inclined surface 12b which comes into contact with a posterior capsule Sb is 1.5 mm, and the thickness gradually reduces from 0.5 mm to 0.2 mm from the radially inward side to the radially outward side. As explained above, by setting the width and thickness of the rear supporting section 12 to be larger than those of the front supporting section 11, or setting the thickness of the rear supporting section 12 to be decreased from the radially inward side to the radially outward side, the rear supporting section 12 can be brought into contact with the posterior capsule Sb widely than the front supporting section 11 is brought into contact with the anterior capsule Sf. Therefore, the rear supporting section 12 can be arranged stably in a lens capsule S.

The connecting section 13 includes, as shown in Fig. 1, four connecting members 131 arranged at equal intervals in the peripheral direction of the front supporting section 11 and the rear supporting section 12. This connecting member 131 is a thin plate-shaped member made of an elastic material such as a synthetic resin, and one end of the connecting member 131 is connected to the rear surface of the front supporting section 11 in a manner as to extend in a right-angle direction or slightly radially outward, and the other end of the connecting member 131 is connected to the front surface of the rear supporting section 12 in a manner as to extend in a right-angle direction or slightly radially outward.

Further, as shown by the solid line in Fig. 3(a), in a natural state in which the connecting members 131 are not elastically deformed, the connecting section 13 arranges the front supporting section 11 and the rear supporting section 12 at a predetermined distance H. This predetermined distance H is a length that causes a slight bending of the connecting members 131 when the present device 1 is arranged in a lens capsule S. Further, as shown by the broken line in Fig. 3(a), when the front supporting section 11 and the rear supporting section 12 move in the approaching direction, this connecting section 13 bends in a manner as to expand radially outward of the front supporting section 11 and the rear supporting section 12. For this reason, when the present device 1 is arranged in a lens capsule S, it becomes a state in which the connecting members 131 is bent radially outward. Utilizing the generated elastic force returning to an original shape, it becomes possible to attain a state in which the connecting section 13 has an urging force in a direction to separate the front supporting section 11 and the rear supporting section 12. Further, this connecting section 13 stretches and extends an anterior capsule Sf and a posterior capsule Sb effectively in the front-back direction around the entire periphery thereof to open a lens capsule equator Se, to thereby provide tonus of moderate strength to a Zinn's zonule Z.

Further, the connecting section 13 is configured to have an urging force corresponding to a tensile force of a Zinn's zonule Z generated at the time of contraction or relaxation of a ciliary muscle Cm of a ciliary body C. With this, when the present device is arranged in a lens capsule S, a tonus of more moderate strength can be continuously given to a Zinn's zonule Z.

Further, as shown in Figs. 3(a) and 3(b), in the connecting section 13, each connecting member 131 is formed to have a bending portion 132 previously formed in a manner as to expand the connecting section 13 radially outward of the front supporting section 11 and the rear supporting section 12. With this, as described later, when the front supporting section 11 and the rear supporting section 12 move in the approaching direction, due to this bending portion 132, the connecting section 13 can bent assuredly in a manner as to expand radially outward. Further, since this bending portion 132 is configured to bend in a bent manner, due to this bending portion 132, the connecting section 13 can more assuredly bend in a manner as to expand radially outward.

Further, in the connecting section 13, the bending portion 132 is formed at a position closer to the rear supporting section 12 than the front supporting section 11 in the connecting member 131. With this, since the front side portion of the connecting member 131 positioned forwardly of the bending portion 132 becomes longer than the rear side portion of the connecting member 131 positioned rearwardly of the bending portion 132 in each connecting member 131, it becomes easy for the front supporting section 11 to move in the front-back direction with respect to the rear supporting section 12. Further, the optical section 141 of the present lens 14 can be arranged more rearward at the time of non-adjustment (at the time of seeing a far distance), which can increase the forward movement at the time of adjustment (at the time of seeing a near distance).

### [Structure of the Lens]

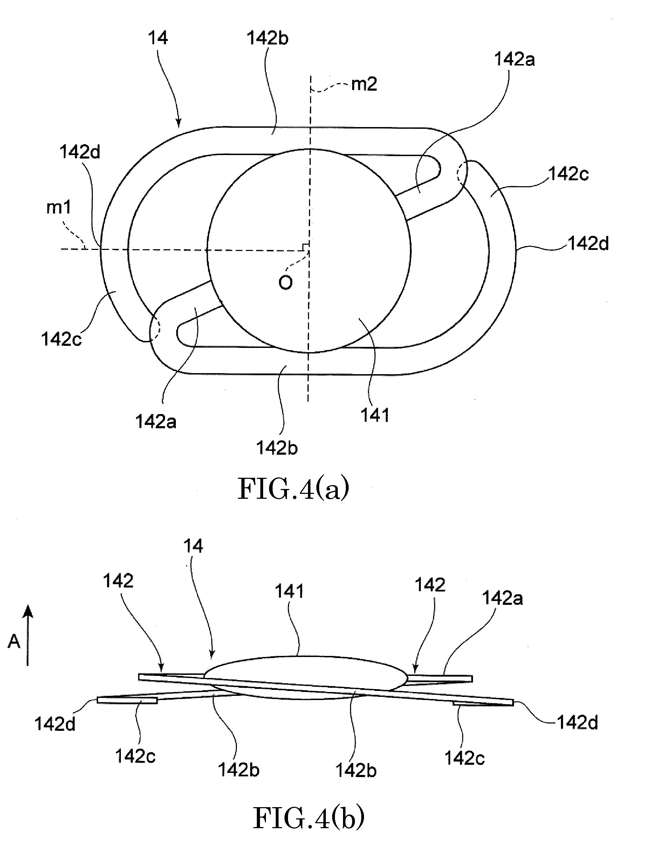

As shown in Fig. 4, the lens 14 includes an optical section 141 made of a convex lens, and two arm sections 142 each connected to the peripheral portion of the optical section 141. Among intraocular lenses, the present lens 14 is called "accommodative intraocular lens" capable of adjusting the focal point by moving the optical section 141 in the front-back direction.

The optical section 141 is a disc-shaped convex lens having a center O, which is made of a synthetic resin material such as silicon, acryl, hydrogel, PMMA, HEMA, hidropolymer, etc.

The arm sections 142 are each made of a flat elastic material made of a synthetic resin such as PMMA, polyimide, acryl, HEMA, polypropylene, etc., and each extends approximately in the plane direction of the optical section 141 so as to be arranged symmetrically with respect to the center O of the optical section 141. Concretely, the arm section 142 includes a connecting portion (one end portion) 142a extending from the peripheral portion of the optical section 141 radially outward in a movable manner, an intermediate portion 142b continuously curved from the tip end of the connecting portion 142a and then linearly extended in a direction toward the peripheral portion of the optical section 141 (in the right direction or left direction in Fig. 4(a)), and a tip end portion (the other end portion) 142c extending from the tip end of the intermediate portion 142b so as to extend along the periphery of the optical section 141 (in the upper direction or lower direction in Fig. 4(a)).

The tip end portion 142c is arranged at a position furthest from the center O among the arm section 142 and also arranged more rearward than the connecting portion 142a. The portion 142d furthest from the center O of the optical section 141 among the tip end portion 142c is a portion which receives a force from the connecting section 13 by being engaged with the connecting section 13 of the present device 1, and therefore will be hereinafter referred to as "support portion 142d."

Further, in the arm section 142, the connecting portion 142a connected to the peripheral portion of the optical section 141 and the tip end portion 142c (support portion 142d) to be engaged with the connecting section 13 are positioned on the opposite sides with respect to the center O of the optical section 141. Concretely, focusing on the left side of the arm section 142 in Fig. 4(a), assuming that a straight line extending from the support portion 142d to the center O of the optical section is defined as a line m1 and a straight line passing the center O of the optical section 141 and orthogonally crossing the line m1 is defined as a line m2, in the right and left regions divided by the line m2, the support portion 142d is positioned on a region opposite to the region where the connecting portion 142a is positioned. Thus, the length from the connecting portion 142a to the support portion 142d can be kept long. The function of this structure will be explained by comparing the present lens 14 which will be explained later with a prior art lens 14'.

In the present lens 14, when an external force is applied to the support portions 142d of the arm sections 142 toward the radially inward side, the optical section 141 will be moved forward. On the other hand, when an external force is applied to the support portion 142d of the arm section 142 toward a radially outward side, the optical section 141 will be moved rearward in a manner as to return to the original position. Further, in cases where the arm section 142 has an elastic force urging outward, when an external force applied to the support portion 142d of the arm section 142 so as to urge radially inward of the optical section 141 is removed, the optical section 141 can be moved rearward in a manner as to return to its original position. Hereinafter, the moving mechanism of the optical section 141 in the front-back direction will be explained concretely.

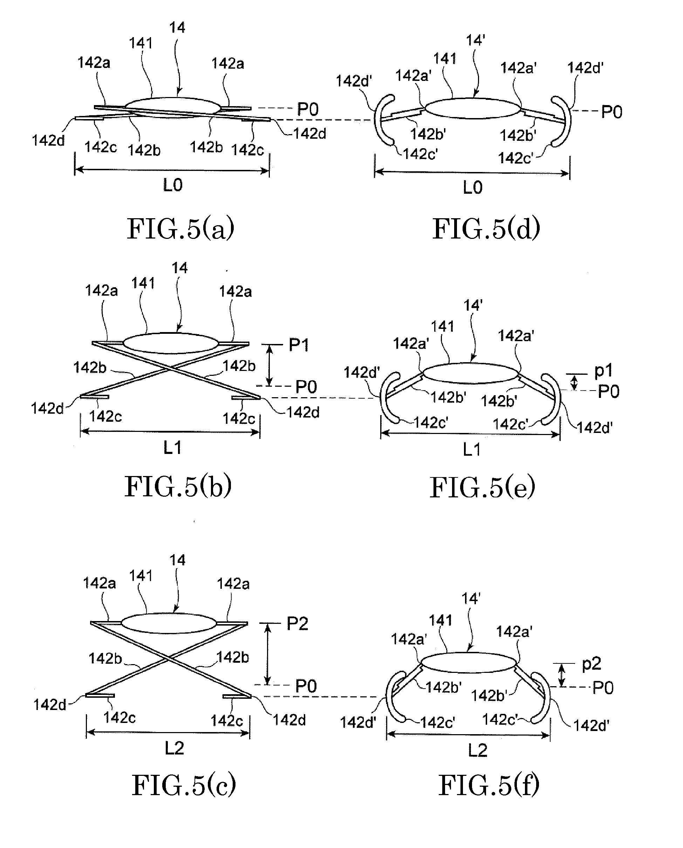

Initially, as shown in Fig. 5(a), each arm section 142 is positioned generally in a plane direction of the optical section 141. At this time, the position of the optical section 141 in the front-back direction is defined as a reference position P0, and the distance between the support portions 142d and 142d of the arm sections 142 and 142 is defined as L0.

As shown in Fig. 5(b), in the present lens 14, when an external force is applied to the support portions 142d and 142d of the arm sections 142 and 142 toward a radially inward side to move both the support portions 142d and 142d in the approaching direction and the distance between both the support portions 142d and 142d becomes L1, each arm section 142 gradually changes its posture from a plane posture to a stereoscopic posture extending in the front-back direction. This is because the force applied to the connecting portion 142a in the plane direction is converted to a force in the front-back direction based on that the arm sections 142 and 142 are structured symmetrically with respect to the optical section 141 and that the tip end portion 142c of the arm section 142 is structured so as to be positioned at the rear side of the connecting portion 142a. With this, the connecting portion 142a is moved forward to the position P1, and in accordance with the movement, the optical section 141 connected to the connecting portions 142a and 142a of both the arm sections 142 and 142 is also moved forward to the position P1.

As shown in Fig. 5(c), in the present lens 14, when an external force is further applied to the support portions 142d and 142d of the arm sections 142 and 142 toward the radially inward side to further move both the support portions 142d and 142d in the approaching direction and the distance between both the support portions 142d and 142d becomes L2, the connecting portions 142a and 142a are further moved forward to the position P2. In accordance with the movement, the optical section 141 can also be further moved forward to the position P2.

On the other hand, in the present lens 14 shown in Fig. 5(c), when an external force is applied radially outwardly to the support portions 142d and 142d of the arm sections 142 and 142 to move both the support portions 142d and 142d in the separating direction and the distance between both the support portions 142d and 142d becomes from L2 to L1, each arm section 142 gradually deforms from the posture extended in the front-back direction to the posture toward the plane direction. Thus, the connecting portions 142a and 142a are moved rearward to the position P1, and in accordance with this movement, the optical section 141 connected to the connecting portions 142a and 142a of both the arm sections 142 and 142 can also be moved rearward to the position P1.

As shown in Fig. 5(a), in the present lens 14, when an external force is further radially outwardly applied to the support portions 142d and 142d of the arm sections 142 and 142 to further move both the support portions 142d and 142d in the separating direction and the distance between both the support portions 142d and 142d becomes from L1 to L0, the connecting portions 142a and 142a are further moved rearward to the position P0. In accordance with the movement, the optical section 141 connected to the connecting portions 142a and 142a of both the arm sections 142 and 142 can also be further moved rearward to the position P0 and returned to its original position.

When compared with an accommodative intraocular lens (see, the accoomodative intraocular lens of the aforementioned Patent Document 1, hereinafter referred to as "prior art lens") in which an arm section has a movable portion for moving an optical section back and forth, the present lens 14 can effectively convert the movements of the support portions 142d and 142d of the arm sections 142 and 142 in the approaching/separating direction into the movements of the optical section 141 in the front-back direction. Hereinafter, this will be explained by comparing the present lens 14 with the prior art lens 14'. In the following explanation, the present lens 14 and the prior art lens 14' include the same optical section 141, and the same numeral/symbol is allocated to the same or corresponding portion. The numeral/symbol in the prior art lens 14 is added by (') dash mark to distinguish from the present lens 14.

In the arm section 142 of the present lens 14, as explained above, the support portion 142d is positioned in the region opposite to the region where the connecting portion 142a is positioned (see Fig. 5(a)). On the other hand, in the arm section 142' of the prior art lens14', the connecting portion 142a' and the support portion 142d' are positioned on the same side (see Fig. 5(d)). For this reason, the length in the present lens 14 from the connecting portion 142a to the support portion 142d is longer than the length in the prior art lens 14' from the connecting portion 142a' and the support portion 142d'.

Based on the above, as shown in Fig. 5(d), in the prior art lens 14', each arm section 142' is positioned approximately in a plane direction of the optical section 141. At this time, the position of the optical section 141 in the front-back direction is defined as a reference position P0, and the distance between the support portions 142d' and 142d' of the arm sections 142' and 142' is L0.

As shown in Fig. 5(e), in the prior art lens 14', when an external force urging radially inward is applied to the support portions 142d' and 142d' of the arm sections 142' and 142' to move both the support portions 142d' and 142d' radially inward and the distance between both the support portions 142d' and 142d' becomes L1, each arm section 142' gradually deforms its posture from the plane direction to the rearward posture extending radially rearward. However, in the prior art lens 14', since the length from the connecting portion 142a' to the support portion 142d' is shorter than in the present lens 14, the connecting portion 142a' moves only from the position P0 to the position p1. Apparent from the comparison of Fig. 5(b) and Fig. 5(e), the position p1 in the prior art lens 14' is positioned rearward than the position P1 in the present lens 14.

As shown in Fig. 5(f), in the prior art lens 14', when an external force is further applied to the support portions 142d' and 142d' of the arm sections 142' and 142' toward the radially inward side to further move both the support portions 142d' and 142d' in the approach direction and the distance between both the support portions 142d' and 142d' becomes L2, the connecting portions 142a' and 142a' are further moved forward to the position p2. In accordance with the movement, the optical section 141 can also be further moved forward to the position p2. However, in the prior art lens 14', the length from the connecting portion 142a' to the support portion 142d' is shorter than in the present lens 14. Therefore, as will be apparent from the comparison of Fig. 5C and Fig. 5(f), the position p2 in the prior art lens14' is rearward than the position P2 in the present lens 14.

On the other hand, in the prior art lens 14' in the state shown in Fig. 5(f), as shown in Fig. 5(e) and Fig. 5(d), when an external force is applied radially outward to the support portions 142d' and 142d' of the arm sections 142' and 142' to move both the support portions 142d' and 142d' in the separating direction, the distance between both the support portions 142d and 142d changes from L2 to L 1 , and L1 to L0. As a result, the optical section 141 moves rearward from the position p2 to the position p1, and then to the position p0, and returns to its original state.

As explained above, in the present lens 14, the length from the connecting portion 142a to the support portion 142d is longer than in the prior art lens 14'. Therefore, the movements of both the support portions 142d and 142d in the approaching/separating direction can be efficiently converted into the movements of the optical section 141 in the front-back direction.

### [Installation and function of the present device and the present lens]

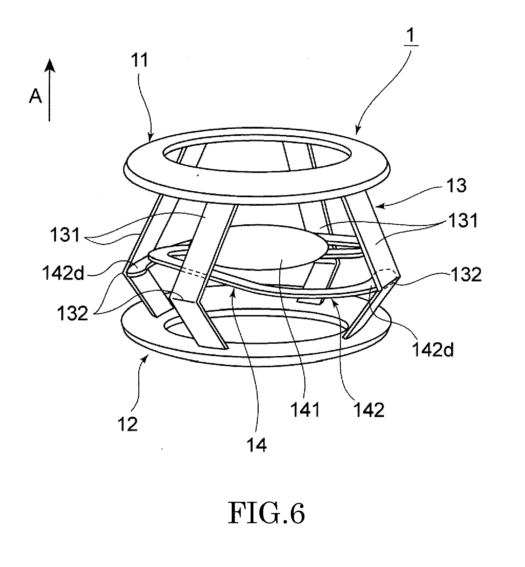

Next, the function of the present device 1 and that of the present lens 14 will be explained with reference to Fig. 6 and Fig. 7.

Initially, in arranging the present lens 14 in the present device 1, as shown in Fig. 6, the present lens 14 is integrally arranged between the front supporting section 11 and the rear supporting section 12 of the present device 1. Concretely, the tip end portions 142c and 142c of the arm sections 142 and 142 are engaged with the bending portions 132 and 132 so that the support portions 142d and 142d of the arm sections 142 and 142 are positioned inside the bending portions 132 and 132 of any opposed two connecting members 131 and 131 in the present device 1. As a result, between the front supporting section 11 and the rear supporting section 12, the optical section 141 of the present lens 14 is arranged in a manner as to be in parallel to the front supporting section 11 and the rear supporting section 12.

In the state in which the present lens 14 is integrally arranged inside the present device 1, when the front supporting section 11 and the rear supporting section 12 move in the approaching direction to increase the degree of radially outward expansion of the connecting section 13, both the support portions 142d and 142d of the present lens 14 move in the separating direction. In accordance with the movement, the optical section 141 of the present lens 14 to which both the connecting portions 142a and 142a of the arm sections 142 and 142 are attached can move rearward between the front supporting section 11 and the rear supporting section 12. Further, when the front supporting section 11 and the rear supporting section 12 move in the separating direction to decrease the degree of radially outward expansion of the connecting section 13, both the support portions 142d and 142d of the arm sections 142 and 142 of the present lens 14 move in the approaching direction. In accordance with the movement, the optical section 141 of the present lens 14 to which both the connecting portions 142a and 142a of the arm sections 142 and 142 are attached can move forward between the front supporting section 11 and the rear supporting section 12.

Further, since the arm sections 142 and 142 of the present lens 14 are engaged with the bending portions 132 and 132 of the present device 1, the present lens 14 can be stably arranged. Further, the bend portion is curved in a bent manner, and therefore the present lens 14 can be arranged more stably.

Next, in arranging the present device 1 in a lens capsule S, as shown in Fig. 7(a), the present device 1 is inserted into a lens capsule S from the cut portion of the anterior capsule Sf in a cataract surgery, etc., so that the present device 1 is arranged in the lens capsule S in parallel therewith in a manner such that the front supporting section 11 is in contact with the inner surface of the anterior capsule Sf and the rear supporting section 12 is in contact with the inner surface of the posterior capsule Sb. At this time, since the distance H between the front supporting section 11 and the rear supporting section 12 is longer than the distance between the anterior capsule Sf and the posterior capsule Sb, the front supporting section 11 and the rear supporting section 12 are moved in the approaching direction by being pressed by the anterior capsule Sf and the posterior capsule Sb. For this reason, each connecting member 131 becomes a bent state in a manner as to expand radially outward due to the bending portion 132. The urging force of each connecting member 131 generated at that time causes the front supporting section 11 to push against the inner surface of the anterior capsule Sf and also causes the rear supporting section 12 to push against the inner surface of the posterior capsule Sb. In order to give a tonus of moderate strength corresponding to the contraction and relaxation of the ciliary muscle Cm of the ciliary body C, it is preferable to provide the present device 1 having a height corresponding to the thickness of the crystalline lens measured by an examination by, e.g., an ultrasonic scan before surgery.

Therefore, the peripheral portion of the lens capsule equator Se is tried to be stretched and extended in the front-back direction, which causes a stretching of the lens capsule equator Se and at the same time a centripetal movement of the lens capsule equator Se. This results in a reduced diameter of the lens capsule equator Se. For this reason, the Zinn's zonule Z is pulled in both directions toward the lens capsule S side and the ciliary body C side, which can apply a continuous tonus of moderate strength to the Zinn's zonule Z. As a result, the Zinn's zonule Z can transmit the slight contraction and relaxation of the ciliary muscle Cm of the ciliary body C to the lens capsule S with a high degree of accuracy, and occurrence of after-cataract can also be prevented.

Further, in accordance with the increase or decrease of the degree of the radially outward expansion of the connecting section 13, the support portions 142d and 142d of the tip end portions 142c move in the separating or approaching direction, which causes movements of the optical section 141 in the front-back direction.

Next, the mechanism of the focusing function by the present device 1 and the present lens 14 installed in a lens capsule S will be explained.

As shown in Fig. 7(a), at the time of seeing a far distance (at the time of non-adjustment), the ciliary muscle Cm of the ciliary body C is relaxed and becomes flat, which results in a retraction of the ciliary body C in a direction separating from the lens capsule S. Thus, by the tensile force of the Zinn's zonule Z having a continuous tonus of moderate strength by the present device 1 inserted in the lens capsule S, the peripheral portion of the lens capsule equator Se is pulled radially outward. As a result, the lens capsule S is deformed so that the thickness of the lens capsule S decreases, and therefore the distance between the anterior capsule Sf and the posterior capsule Sb becomes short. Therefore, the front supporting section 11 and the rear supporting section 12 move each other in the approaching direction. For this reason, each connecting member 131 becomes a bent state in a manner as to expand radially outward due to the bending portion 132. The urging force of each connecting member 131 generated at that time causes the front supporting section 11 to push against the inner surface of the anterior capsule Sf and also causes the rear supporting section 12 to push against the inner surface of the posterior capsule Sb, which becomes a state in which it is balanced with the tensile force of the Zinn's zonule Z. At this time, the degree of radially outward expansion of the connecting section 13 increases, and therefore both the support portions 142d and 142d respectively move in the separating direction to thereby increase the distance between both the support portions 142d and 142d. As a result, the optical section 141 moves backward. Thus, depending on the relaxation of the ciliary muscle Cm of the ciliary body C, the adjustment function of the present lens 14 can be exerted at the time of seeing a far distance.

On the other hand, at the time of seeing a near object (at the time of adjustment), as shown in Fig. 7(b), the ciliary muscle Cm of the ciliary body C contracts to protrude centripetally (toward the lens capsule S side), which decreases the degree of tonus of the Zinn's zonule Z. As a result, the tonus of the peripheral portion of the lens capsule equator Se is loosened, and therefore the front supporting section 11 and the rear supporting section 12 are urged by the urging force by the connecting section 13 and moved in the separating direction while resisting the tensile force of the Zinn's zonule Z. At this time, the connecting members 131 elastically deform toward its original state. Thus, the outwardly bent degree of the connecting members 131 decreases, causing both the support portions 142d and 142d to move in the approaching direction to reduce the distance between both the support portions 142d and 142d. This moves the optical section 141 forward. As explained above, depending on the contraction of the ciliary muscle Cm of the ciliary body C, the adjustment function of the present lens 14 can be exerted at the time of seeing a near distance.

As will be understood from the above, due to the structure of the present device 1, the Zinn's zonule Z can accurately transmits slight contraction and relaxation of the ciliary muscle Cm of the ciliary body C to the lens capsule S, which in turn can accurately exert the adjustment function of the present lens 14.

In the present device 1, the present device is not arranged in a manner as to be in contact with the lens capsule equator Se. The lens capsule equator Se is expanded by the urging force of the plurality of connecting members 131, so that the lens capsule equator Se is exposed to hydatoid. This inhibits growth and fibrillization of the lens epidermal cell at the lens capsule equator Se, which can prevent occurrence of after-cataract. Especially, in this embodiment, hydatoid flows into through the front supporting section 11 formed circularly and assuredly reaches the lens capsule equator Se between the connecting members 131, which assuredly inhibits growth and fibrillization of a lens epidermal cell in a lens capsule equator Se.

Further, by utilizing the present device 1, the present lens 14 can be stably arranged in the lens capsule S.

Further, in the present lens 14 according to this embodiment, as shown in Fig. 4, the length from the connecting portion 142a to the support portion 142d is longer than in the prior art lens 14'. Therefore, movements of both the support portions 142d and 142d in the approaching/separating direction can be efficiently converted into movements of the optical section 141 in the front-back direction. As a result, even in the case of a slight movement of the lens capsule S due to slight contraction and relaxation of the ciliary muscle Cm of the ciliary body C, it is possible to effectively exert the adjustment function of the intraocular lens 14.

That is, the contraction and relaxation of the ciliary muscle Cm of the ciliary body C is slight, and therefore the amount of change of the lens capsule S via the Zinn's zonule Z is also small. For this reason, the movement of the anterior capsule Sf and the posterior capsule Sb in the approaching/separating direction is small, and therefore the amount of movement of the front supporting section 11 and the rear supporting section 12 in the approaching/separating direction is also small. However, as explained above using Fig. 5, in the present lens 14, since the length from the connecting portion 142a of the arm section 142 to the support portion 142d of the tip end portion 142c is long, even if the amount of movement of the front supporting section 11 and the rear supporting section 12 in the approaching/separating direction is small, the small movement of both the support portion 142d and 142d in the approaching/separating direction can be effectively converted to a large movement of the optical section 141 in the front-back direction. This enables effective exertion of the adjustment function of the intraocular lens 14.

In the present embodiment, the above explanation was directed to the case in which the present device 1 has four connecting members 131, but not limited to it. The present device 1 can have any number of connecting members.

Further, the above explanation was directed to the case in which the connecting section 13 includes connecting members 131 arranged at equal intervals in the peripheral direction of the front supporting section 11 and the rear supporting section 12, but not limited to such equal interval arrangement.

Further, the explanation was directed to the case in which the connecting section 13 bends so that the connecting section 13 is bent at a portion, but can bend in a manner as to form a gentle curve.

Further, the above explanation was directed to the case in which the connecting section 13 includes a previously formed bending portion 132, but the connecting section 13 can be configured such that the connecting section 13 has no previously formed bending portion 132 and bends or curves only when a force is applied in the direction that the front supporting section 11 and the rear supporting section 12 approach.

Further, the above explanation was directed to the case in which the connecting section 13 includes a bending portion 132 provided at the position closer to the rear supporting section than the front supporting section 11, but not limited to it.

Further, the explanation was directed to the case in which the connecting section 13 is formed by a thin plate-shaped connecting members 131, but the connecting member can be of any shape.

Further, when the front supporting section 11 and the rear supporting section 12 move in the approaching direction, the bending portions 132 bend in a manner as to expand radially outward of the front supporting section 11 and the rear supporting section 12. But, the connecting section 13 is not limited to it, but can be a member which does not bend, but linearly deforms in the front-back direction like a coil spring in a manner as to have an urging force.

Further, the above explanation was directed to the case in which the connecting section 13 is formed by connecting members 131 which are all formed by an elastic material, but at least two connecting members 131 for engaging the present lens 14 are not always required to be formed by an elastic member.

Further, the above explanation was directed to the case in which the front supporting section 11 and the rear supporting section 12 are each formed into a circular or ring shape, but can be formed into any shape.

Further, the above explanation was directed to the case in which before installing the present device 1 in a lens capsule S, the present lens 14 is integrally arranged in the present device 1, but the present lens 14 can be arranged in the present device 1 after installing the present device 1 in a lens capsule S. In installing the present device 1, a method of inserting the present device 1 loaded in an injector in a folded manner into a lens capsule S, a method of inserting the present device 1 into a lens capsule S with the present device 1 folded with tweezers, etc., can be exemplified.

Further, the above explanation was directed to the case in which in the intraocular lens 14, the arm section 142 is a thin plate-shaped member, but can be of any shape.

Further, the above explanation was directed to the intraocular lens 14 in which the connecting portion 142a and the support portion 142d of the arm section 142 are positioned on opposite sides with respect to the center O of the optical section 141, but can be positioned on the same side.

Further, the above explanation was directed to the case in which the present lens explained above is arranged in the present device , but any intraocular lens other than the present lens can be installed, or only the present device can be installed for preventing a possible after-cataract.

### <Second Embodiment>

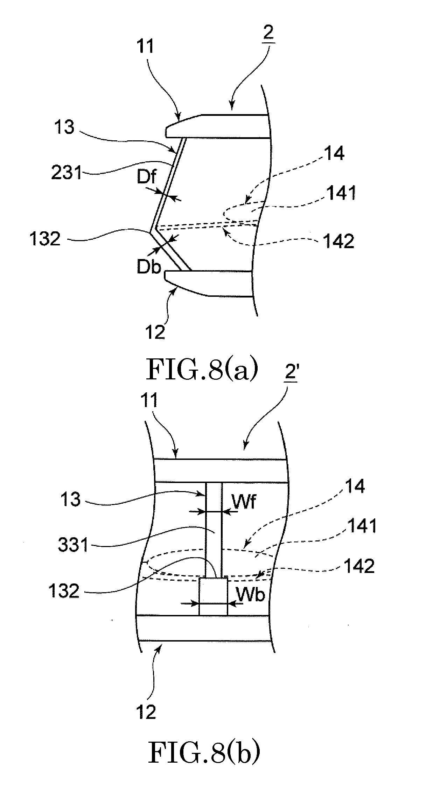

Next, a second embodiment of a lenticular capsule-expanding device according to the present invention will be explained with reference to Fig. 8. Hereinafter, the following explanation will be directed to the structure different from that of the aforementioned embodiment, and the explanation of the same structure will be omitted by allotting the same reference numeral or symbol.

In the present device 2 according to this embodiment, the connecting section 13 is difference in thickness and width at the front side and the rear side with respect to the bending portion 132.

For example, in the connecting section 13, as shown in Fig. 8(a), the thickness Df of the front side portion of the connecting member 231 arranged on the front side of the bending portion 132 is made thinner than the thickness Db of the rear side portion of the connecting member 231 arranged on the rear side of the bending portion 132.

Further, in the present device 2' according to a modification of the example, in the connecting section 13, as shown in Fig. 8(b), the width Wf of the front side portion of the connecting member 331 arranged on the front side of the bending portion 132 is made thinner than the width Wb of the rear side portion of the connecting member 331 arranged on the rear side of the bending portion 132.

In the aforementioned two examples, the explanation was directed to the case in which either one of the thickness and the width of the connecting section is different at the bending portion, but it can be configured such that both of the thickness and the width are different.

### <Third Embodiment>

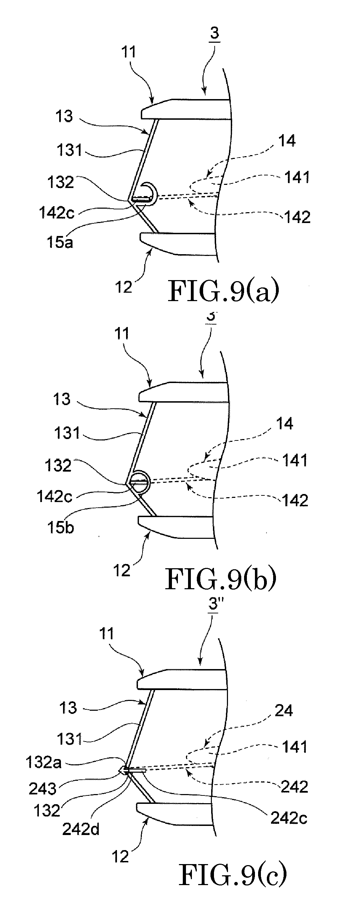

Next, a third embodiment of a lenticular capsule-expanding device according to the present invention will be explained with reference to Fig. 9. Hereinafter, the following explanation will be directed to the structure different from that of the aforementioned embodiments, and the explanation of the same structure will be omitted by allotting the same reference numeral or symbol.

In the present device 3 according to this embodiment, as shown in Fig. 9(a), the connecting section 13 is provided with an engaging portion 15a for engaging the arm section 142 of the present lens 14 on the inner side of the bending portion 132. Concretely, this engaging portion 15a is protruded radially inward from the slightly rearward portion of the inner side of the bending portion 132, and is an elastic member having a tip end curved forward in an arc-shaped manner. With this structure, by forcibly inserting the tip end portion 142c of the arm section 142 into between the tip end portion of the engaging portion 15a and the connecting member 131, the tip end portion 142c of the arm section 142 can be engaged with the bending portion 132.

Further, as the present device 3' according to a modified example, as shown in Fig. 9(b), the engaging portion may be an engaging member 15b having an insertion hole extending along the peripheral direction of the front supporting section 11 and the rear supporting section 12 at the inner side of the bending portion 132. With this structure, by inserting the tip end portion 142c of the arm section 142 of the present lens 14 into the insertion hole of the engaging member 15b, the tip end portion 142c of the arm section 142 can be engaged with the bending portion 132.

Further, as the present device 3" according to a further modified example, as shown in Fig. 9(c), the engaging portion may be an insertion hole 132a extended in the radial direction at the inner side of the bending portion 132. In this case, it is preferable that the present lens 24 is provided with a hook portion 243 formed on the radially outside of the support portion 242d of the arm section 242. With this, by inserting the hook portion 243 of the present lens 24 into the insertion hole 132a of the present device 3" to be hooked therewith, the tip end portion 242c of the arm section 242 can be engaged with the bending portion 132.

By engaging the tip end portion 142c (242c) of the arm section 142 of the present lens 14 with the bending portion 132 of the connecting section 13 by the engaging portion 15a (15b, 132a) as explained above, the present lens 14 can be arranged in the present device 3 (3', 3") more stably and easily.

In the aforementioned embodiments, the engaging portion for engaging the present lens 14 is provided, but it can be configured such that without providing an engaging portion, the present lens 14 can be fixed to the inner side of the connecting section 13 by the elastic force urging radially outward of the arm section 142. In this case, however, the elastic force urging radially outward of the arm section 142 of the present lens 14 should be rather smaller than the elastic force (urging force) of the connecting section 13 of the present device 1.

### <Fourth Embodiment>

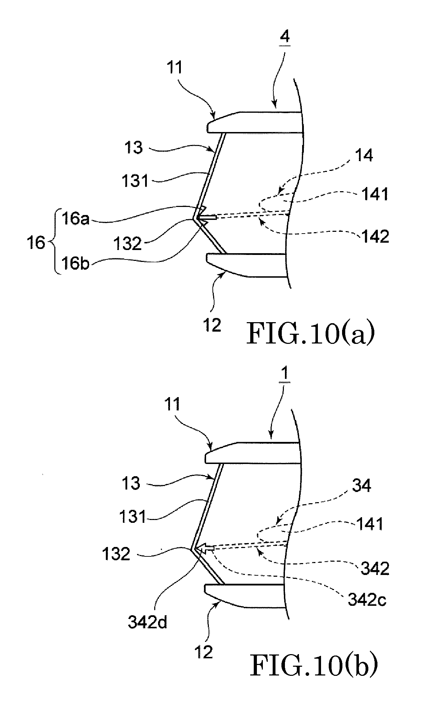

Next, a fourth embodiment of a lenticular capsule-expanding device according to the present invention will be explained with reference to Fig. 10. Hereinafter, the following explanation will be directed to the structure different from that of the aforementioned embodiments, and the explanation of the same structure will be omitted by allotting the same reference numeral or symbol.

In the present device 4 according to this embodiment, as shown in Fig. 10(a), the connecting section 13 is provided with a control portion 16 for controlling the bending of the bending portion 132 so as not to exceed a predetermined degree (angle). Concretely, this control portion 16 includes a front side control section 16a in which the thickness gradually increases from the inner side position of the bending portion 132 toward the front side and a rear side control section 16b in which the thickness increases from the inner side position of the bending portion 132 toward the rear side. As explained above, by controlling the bending of the bending portion 132 so as not to exceed the predetermined degree (angle) by providing the front side control section 16a and rear side control section 16b of the control portion 16 on the inner side of the connecting section 13, the front supporting section 11 and the rear supporting section 12 can be kept at a predetermined distance. This prevents adhesion of the lens capsule equator Se and its peripheral part, which in turn can more assuredly inhibit growth and fibrillization of a lens epidermal cell in a lens capsule equator Se after surgery.

In this embodiment, the explanation was directed to the case in which the control portion 16 is provided with the front side control section 16a and the rear side control section 16b, but the control portion can have another structure such as a cut-out portion, etc.

Further, the control portion can be provided at the present lens. Concretely, as shown in Fig. 10(b), in the present lens 34, the tip end portion 342c (support portion 342d) of the arm section 342 is formed in a manner such that the thickness of the cross-section gradually increases from the radially outside toward the radially inside. As explained above, the tip end portion 342c of the arm section 342 is formed, and therefore the bending degree of the connecting member 131 of the connecting section 13 can be controlled. Therefore, the front supporting section 11 and the rear supporting section 12 are maintained in a state in which both the sections are kept at a constant distance. As a result, in the same manner as in the aforementioned examples, this prevents adhesion of the lens capsule equator Se and its peripheral part, which in turn can more assuredly inhibit growth and fibrillization of the lens epidermal cell in the lens capsule equator Se after surgery.

### <Fifth Embodiment>

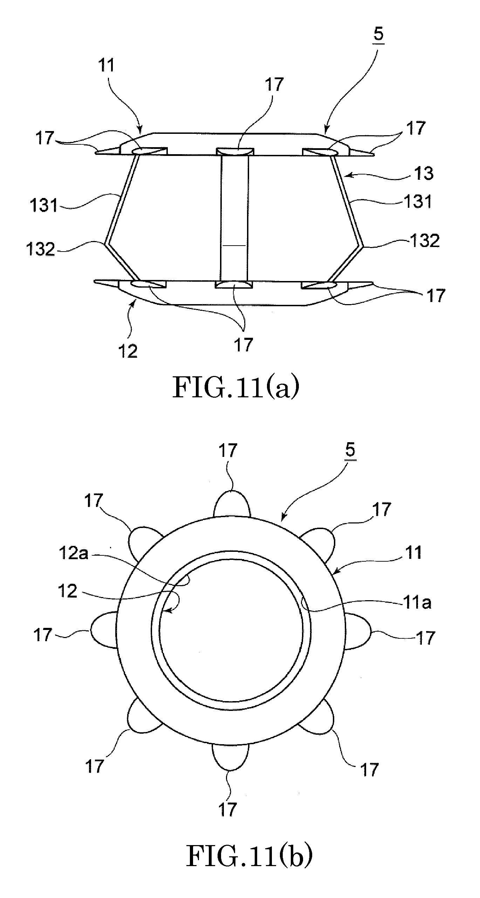

Next, a fifth embodiment of a lenticular capsule-expanding device according to the present invention will be explained with reference to Fig. 11. Hereinafter, the following explanation will be directed to the structure different from that of the aforementioned embodiments, and the explanation of the same structure will be omitted by allotting the same reference numeral or symbol.

In the present device 5 of this embodiment, as shown in Fig. 11, the front supporting section 11 and the rear supporting section 12 are each provided with a plurality of elastic protrusions 17 each protruded radially outward in the radial fashion from the outer peripheral portion thereof. This elastic protrusions 17 are each formed into a thin plate shape (flat shape) in the peripheral direction of the front supporting section 11 and the rear supporting section 12. A total of eight elastic protrusions are arranged at equal intervals in the peripheral direction so as to come into contact with an inner surface of an anterior capsule Sf and an inner surface of a posterior capsule Sb of a lens capsule S when the present device 5 is arranged in a lens capsule S. By this elastic protrusion 17, a peripheral portion of a lens capsule equator Se is further stretched and expanded in the front-back direction to thereby further expand a lens capsule equator Se, which in turn can more assuredly inhibit growth and fibrillization of the lens epidermal cell in the lens capsule equator Se after surgery.

In this embodiment, the explanation was directed to the case in which the elastic protrusions 17 are provided at both the front supporting section 11 and the rear supporting section 12, but the elastic protrusion 17 can be provided at either one of the front supporting section 11 and the rear supporting section 12.

Further, the explanation was directed to the case in which eight elastic protrusions 17 are provided at each of the front supporting section 11 and the rear supporting section 12. But, the number of the elastic protrusions 17 can be arbitrarily selected, and the elastic protrusions 17 are not required to be arranged at equal intervals.

Further, the explanation was directed to the case in which the elastic protrusion 17 is formed into a thin plate shape, but the elastic protrusion can be of any shape.

### <Sixth Embodiment>

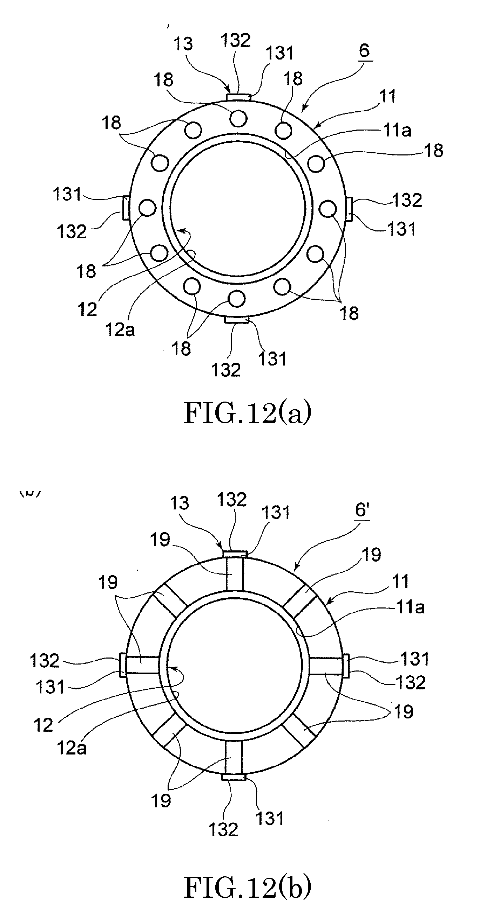

Next, a sixth embodiment of a lenticular capsule-expanding device according to the present invention will be explained with reference to Fig. 12. Hereinafter, the following explanation will be directed to the structure different from that of the aforementioned embodiments, and the explanation of the same structure will be omitted by allotting the same reference numeral or symbol.

In the present device 6 of this embodiment, as shown in Fig. 12(a), the front supporting section 11 and the rear supporting section 12 are each provided with a total of eight thorough-holes 18 each extending in the back-and forth direction and arranged at regular intervals in the peripheral direction at portions which come into contact with an anterior capsule Sf and a posterior capsule Sb. With this, hydatoid flows to the portion where the front supporting section 11 and an anterior capsule Sf contact and the portion where the anterior capsule Sf and a posterior capsule Sb contact via the through-holes 18. This inhibits growth of the lens epidermal cell at the contact portion to thereby prevent occurrence of after-cataract and also prevent anterior capsule contraction occurred followed by after-cataract.

In this embodiment, the above explanation was directed to the case in which twelve through-holes 18 are provided. But, the number of the through-holes 18 can be arbitrarily selected, and the through-holes 18 are not required to be arranged at equal intervals.

Further, the above explanation was directed to the case in which the through-holes 18 are provided at both the front supporting section 11 and the rear supporting section 12, but the through-holes 18 can be provided at either one of the front supporting section 11 and the rear supporting section 12.

Further, as shown in Fig. 12(b), as the present device 6' according to a modified example, in place of the through-hole 18, a groove 19 may be formed in a radial fashion.

For this reason, even in the case of providing the groove 19, hydatoid can be introduced to the portion where the front supporting section 11 and an anterior capsule Sf contact.

Further, the above explanation was directed to the case in which one of the through-hole 18 and the groove 19 is formed on the front supporting section 11 and/or the rear supporting section 12, but both the through-hole 18 and the groove 19 may be provided at the front supporting section 11 and/or the rear supporting section 12.

### <Seventh Embodiment>

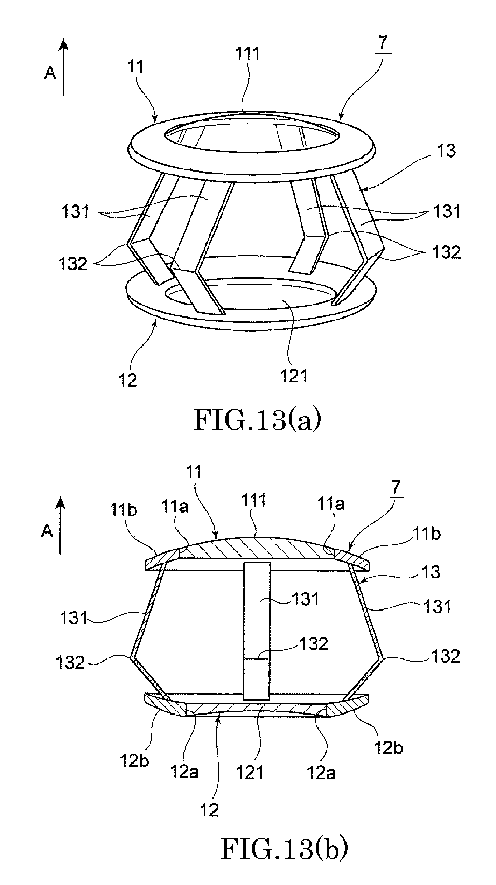

Next, a seventh embodiment of a lenticular capsule-expanding device according to the present invention will be explained with reference to Fig. 13. Hereinafter, the following explanation will be directed to the structure different from that of the aforementioned embodiments, and the explanation of the same structure will be omitted by allotting the same reference numeral or symbol.

In the present device 7 according to this embodiment, as shown in Fig. 13, the front supporting section 11 is provided with a transparent convex first corrective lens 111 in a manner as to close the opening 11a. This first corrective lens 111 is to correct the power of the optical section 141 of the intraocular lens 14 to be arranged inside, and the power of the first corrective lens 111 is set depending on the power of the optical section 141. The front supporting section 11 is formed into a ring shape, and the first corrective lens 111 arranged in the opening 11a is transparent, and therefore light can pass through toward the rear side.

Further, the rear supporting section 12 is provided with a transparent concave second corrective lens 121 in a manner as to close the opening 12a. This second corrective lens 121 is to correct the power of the optical section 141 of the intraocular lens 14 which will be explained later to increase the range of the power of the first corrective lens 111. For this reason, the power of the second corrective lens 121 is set depending on the power of the optical section 141 or the power of the first corrective lens 111. The rear supporting section 12 is formed into a ring shape, and the second corrective lens 121 arranged in the opening 12a is transparent, and therefore light can pass through toward the rear side. For this reason, when the present device 7 is arranged in a lens capsule S, the light passed the front supporting section 11 and the rear supporting section 12 can reach a retina.

As explained above, in the front supporting section 11, it is structured such that the first corrective lens 111 is detachably attached. Therefore, the first corrective lens 111 can be arbitrarily attached or replaced with another lens. Further, also in the rear supporting section 12, it is structured such that the second corrective lens 121 can be detachably attached. Therefore, the second corrective lens 121 can be arbitrarily attached or replaced with another lens.

In this embodiment, the above explanation was directed to the case in which the first corrective lens 111 and the second corrective lens 121 are provided, but either one of the first corrective lens 111 and the second corrective lens 121 can be provided.

Further, the above explanation was directed to the case in which the first corrective lens 111 and the second corrective lens 121 are detachably attached to the front supporting section 11 and the rear supporting section 12, but it can be structured such that the first corrective lens 111 and the second corrective lens 121 are integrally attached to the front supporting section 11 and the rear supporting section 12, respectively.

### <Eight Embodiment>

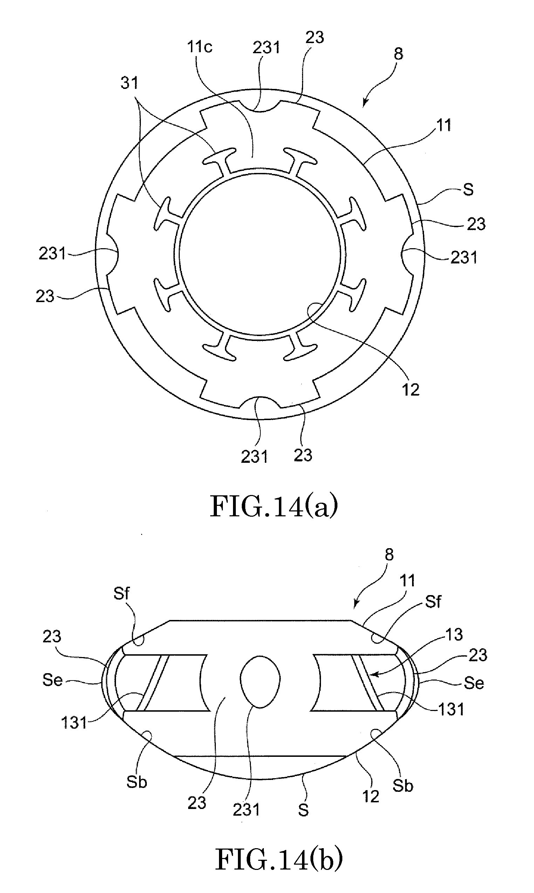

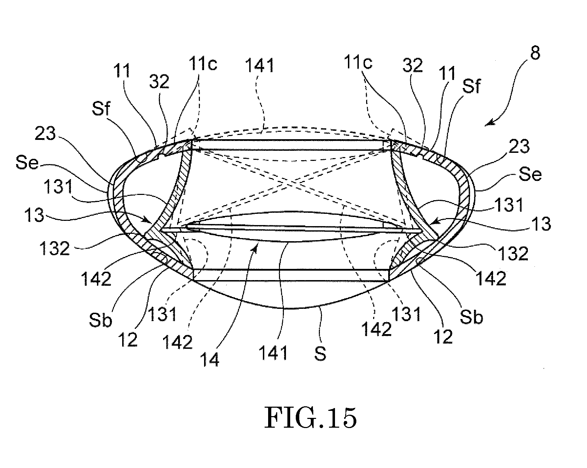

Next, an eighth embodiment of a lenticular capsule-expanding device according to the present invention will be explained with reference to Figs. 14(a), 14(b) and 15. Hereinafter, the following explanation will be directed to the structure different from that of the aforementioned embodiments, and the explanation of the same structure will be omitted by allotting the same reference numeral or symbol.

In the present device 8 according to this embodiment, as shown in Fig. 14(a), the front supporting section 11 is provided with a plurality of cut-out portions 31 extending radially outward from the inner peripheral portion 11c. With this, the inner peripheral portion 11c of the front supporting section 11 becomes more easily movable in response to movements of an inner peripheral portion of a crystalline lens anterior capsule Sf which most moves by adjustment. For this reason, like in this embodiment, when the connecting section 13 is connected to the inner peripheral portion 11c of the front supporting section, as shown by the dot line in Fig. 15, in accordance with the movement of the inner peripheral portion 11c of the front supporting section 11, the bending degree of the connecting section 13 also changes largely. Therefore, it becomes possible to largely move the optical section 141 of the intraocular lens 14 between the front supporting section 11 and the rear supporting section 12 in the front-back direction. In this embodiment, at the position slightly radially outward than the inner peripheral portion 11c on the rear surface of the front supporting section 11, a groove 32 extending in the peripheral direction is formed, so that the inner peripheral portion 11c of the front supporting section 11 can be further easily moved.

Further, at the radially outward positon of the connecting section 13 of the present device 8, a total of four second connecting sections 23 connecting the front supporting section 11 and the rear supporting section 12 are provided. These second connecting sections 23 are arranged along the peripheral direction of the present device 8 at equal intervals, and have an urging force for separating the front supporting section 11 and the rear supporting section 12 in the separating direction. Further, the second connecting section 23 is formed into a wide shape extending along an inner peripheral surface of an equator Se of a lens capsule S, and has, at its central portion, an approximately elliptically-shaped hole 232 for enhancing passing of hydatoid.

According to this, together with or in place of the connecting section 13 used to mainly move the intraocular lens 14, the front supporting section 11 and the rear supporting section 12 can press against the anterior capsule Sf and the posterior capsule Sb by the urging force of the second connecting section 23. Further, the second connecting section 23 is formed into a shape extending along an inner peripheral surface of an equator Se of a lens capsule S, and therefore the present device 8 can be stably arranged in the lens capsule S.

Further, since hydatoid can easily flow to an equator Se of a lens capsule S via the holes of the connecting sections 23, occurrence of after-cataract can be easily inhibited.

Although several embodiments of the present invention have been explained with reference to drawings, the present invention is not limited to the illustrated embodiments. Within the same scope as the present invention or its equivalent range, various changes or modifications can be made to the illustrated embodiments,.

## Claims

1. A lenticular capsule-expanding device to be arranged in a lens capsule in which an anterior capsule is cut in a cataract surgery, etc., comprising:
a front supporting section to be arranged in a manner as to come into contact with an inner surface of the anterior capsule, the front supporting section allowing light to pass through rearward;
a rear supporting section to be arranged in a manner as to come into contact with an inner surface of a posterior capsule, the rear supporting section being arranged so as to be opposed to the front supporting section and allowing light to pass through rearward; and
a connecting section that connects the front supporting section and the rear supporting section in a manner as to have an urging force for separating the front supporting section and the rear supporting section in a separating direction,
wherein, by the urging force of the connecting section, the front supporting section presses against the inner surface of the anterior capsule and the rear supporting section presses against the inner surface of the posterior capsule.

2. The lenticular capsule-expanding device as recited in claim 1,
wherein the connecting section has an urging force corresponding to a tensile force of a Zinn's zonule to be generated at a time of contraction or relaxation of a ciliary muscle of a ciliary body.

3. The lenticular capsule-expanding device as recited in claim 1,
wherein the connecting section includes a plurality of connecting pieces arranged at intervals in a peripheral direction of the front supporting section and the rear supporting section.

4. The lenticular capsule-expanding device as recited in claim 1,
wherein the connecting section bends radially outward of the front supporting section and the rear supporting section when the front supporting section and the rear supporting section move in an approaching direction.

5. The lenticular capsule-expanding device as recited in claim 4,
wherein the connecting section includes a previously formed bent portion capable of being bent radially outward of the front supporting section and the rear supporting section.

6. The lenticular capsule-expanding device as recited in claim 5,
wherein the connecting section is capable of being bent in a manner such that the bent portion bends.

7. The lenticular capsule-expanding device as recited in claim 5,
wherein the bent portion of the connecting section is provided at a position closer to the rear supporting section than the front supporting section.

8. The lenticular capsule-expanding device as recited in claim 5,
wherein the connecting section is provided with an engaging portion for engaging an intraocular lens at an inner side of the bent portion.

9. The lenticular capsule-expanding device as recited in claim 5,
wherein the connecting section is provided with a control portion for controlling bending of the bent portion exceeding a predetermined degree of bending.

10. The lenticular capsule-expanding device as recited in claim 1,
wherein the front supporting section and/or the rear supporting section is provided with a plurality of elastic protrusions protruded radially outward of an outer peripheral portion in a radial fashion.

11. The lenticular capsule-expanding device as recited in claim 1,
wherein the front supporting section and/or the rear supporting section is provided with at least one of a groove and a through-hole at a portion which comes into contact with an anterior capsule of a crystalline lens.

12. The lenticular capsule-expanding device as recited in claim 1,
wherein at least one of the front supporting section and the rear supporting section is formed into a ring shape having an opening at its center.

13. The lenticular capsule-expanding device as recited in claim 12,
wherein the front supporting section is provided with a plurality of cut-out portions extending radially outward from an inner peripheral portion in a radial fashion.

14. The lenticular capsule-expanding device as recited in claim 1, further comprising a second connecting section which connects the front supporting section and the rear supporting section in a manner as to have an urging force for separating the front supporting section and the rear supporting section in a separating direction, the second connecting section being formed into a shape along an inner peripheral surface of an equator of a lens capsule,
wherein, by the urging force of the second connecting section, the front supporting section presses against the inner surface of the anterior capsule and the rear supporting section presses against the inner surface of the posterior capsule.

15. The lenticular capsule-expanding device as recited in claim 1,
wherein an intraocular lens is arranged between the front supporting section and the rear supporting section.

16. The lenticular capsule-expanding device as recited in claim 15,
wherein the intraocular lens includes an optical section made of a lens and a plurality of arm portions provided at a peripheral portion of the optical section,
wherein each arm portion is movably connected to a peripheral portion of the optical section, and the other end portion is engaged with the connecting section, and
wherein when the other end portion of the arm portion is moved in the approaching/separating direction depending on a degree of expansion of the connecting section radially outward, the optical section to which the one end portion of the arm portion is connected moves between the front supporting section and the rear supporting section in a front-back direction.

17. The lenticular capsule-expanding device as recited in claim 16,
wherein the one end portion of the arm portion connected to a peripheral portion of the optical section and the other end portion of the arm portion to be engaged with the connecting section are arranged on opposite sides with respect to a center of the optical section.
